# EUROPÄISCHE PATENTSCHRIFT

(11) **EP 2 303 138 B1**
(45) Veröffentlichungstag und Bekanntmachung des Hinweises auf die Patenterteilung: **31.08.2011**
(21) Anmeldenummer: 09753857.3
(22) Anmeldetag: 25.05.2009
(51) Int. Cl.: A61B 10/00, B01L 3/00, G01N 1/28, A61B 19/00

(54) **ANORDNUNG ZUM TRANSPORT UND/ODER ZUR AUFBEWAHRUNG EINER MENSCHLICHEN ODER TIERISCHEN GEWEBEPROBE**
ARRANGEMENT FOR TRANSPORT AND/OR SAFEKEEPING OF A HUMAN OR ANIMAL TISSUE SAMPLE
ENSEMBLE DE TRANSPORT ET/OU DE CONSERVATION D'UN ÉCHANTILLON TISSULAIRE HUMAIN OU ANIMAL

(30) Priorität: 31.05.2008 DE 202008007356 U; 05.08.2008 DE 202008010411 U
(43) Veröffentlichungstag der Anmeldung: 06.04.2011
(73) Patentinhaber: Klinika Medical GmbH, 61250 Usingen (DE)
(72) Erfinder: KITTA, Johann Karl, 51545 Waldbröl-Herfen (DE); HUSCHMAND NIA, Abdolhamid, 66953 Pirmasens (DE)
(74) Vertreter: Haverkamp, Jens
(86) Internationale Anmeldenummer: PCT/EP2009/056320
(87) Internationale Veröffentlichungsnummer: WO 2009/144202

(56) Entgegenhaltungen:
- WO-A1-00/49447
- WO-A2-02/059571
- WO-A2-2006/092797
- DE-U1-202009 013 353
- US-A- 5 002 735

## Beschreibung

Gegenstand der Erfindung ist eine Anordnung für den Transport und/oder die Aufbewahrung einer menschlichen oder tierischen Gewebeprobe, umfassend ein Tablett und ein oder mehrere zur Fixierung der Gewebeprobe auf dem Tablett dienende, jeweils eine Spitze aufweisende und mit dieser in das Tablett einstechbare Fixierstifte.

Chirurgisch einem menschlichen oder tierischen Körper entnommenes Gewebe, beispielsweise ein Tumor wird als Gewebeprobe in aller Regel weiteren Untersuchungen unterzogen. Derartige feingewebliche Untersuchungen werden von einem Pathologen vorgenommen. Handelt es sich bei einer solchen Gewebeprobe um einen Tumor, ist es insbesondere bei einem bösartigen Tumor wichtig, im Wege einer solchen Untersuchung in Erfahrung zu bringen, ob der Tumor tatsächlich vollständig entfernt worden ist. Dieses ist der Fall, wenn ein Sicherheitssaum von gesundem Gewebe um den entfernten Tumor vorhanden ist. Ist ein solcher Tumor nicht oder nicht vollständig und somit nicht mit einem ausreichenden Sicherheitssaum entfernt worden, braucht der Chirurg zudem die Information, welche Seite des Tumors keinen ausreichenden Sicherheitssaum trägt, damit an dieser Stelle eine Nachresektion erfolgen kann. Zu diesem Zweck ist es erforderlich, dass die Orientierung der entnommenen Gewebeprobe innerhalb des Körpers dokumentiert ist und bleibt.

Derzeitig werden unterschiedliche Methoden angewandt, um entnommene Gewebeprobe hinsichtlich ihrer Orientierung im Körper zu kennzeichnen. Dieses erfolgt mitunter durch Einbringen diverser Fäden in die Gewebeprobe, um zu markieren, welcher Seite des entnommene Gewebes welcher Orientierung im Körper entspricht. Auch werden mitunter Farbmarkierungen an der Probe selbst angebracht. Abgesehen davon, dass derartige Markierungen unzureichend sind, da ein zuverlässige Orientierung nicht möglich ist, hat das Markieren der Probe selbst zudem den Nachteil, dass sich derartige Markierungen im Röntgenbild erkennbar sein und somit die Diagnose beeinträchtigen können. Dieses ist unerwünscht, da eine solche Abbildungsüberlagerung eine Diagnose beeinträchtigen oder gar verfälschen kann. Bei einer Kennzeichnung der Gewebeproben selbst ist noch Sorge für einen Transport und/oder eine Aufbewahrung der Gewebeprobe zu tragen.

Gemäß einer anderen Ausgestaltung zum Transportieren von Gewebeproben werden Korkplatten verwendet, auf denen die entnommenen Gewebeproben mit den ohnehin in einem Operationssaal zur Verfügung stehenden Stahlkanülen befestigt werden. Die Stahlkanülen werden durch die entnommene Gewebeprobe hindurchgestoßen und in der Korkplatte festgesetzt. Anschließend wird die Korkplatte sichtlich der Orientierung der entnommenen Gewebeprobe im Körper beschriftet. Die Verwendung von Stahlkanülen ist nicht unproblematisch. Derartige Stahlkanülen verfügen über eine sehr scharfe Spitze, so dass für das eine solche Probe handhabende Personal eine nicht unerhebliche Verletzungs- und insbesondere Infektionsgefahr besteht. Die Kanülenspitze kann aus der Unterseite der Korkplatte herausragen, was nicht selten ist. Zudem kann in dem Kanal der Kanüle Material der Gewebeprobe enthalten sein. Überdies besteht die Gefahr bei derartigen Stahlkanülen, dass beim Transport der Gewebeprobe diese aus dem Korb herausrutschen und damit die Orientierung der Gewebeprobe verloren geht. Die Gewebeprobe muss, um ein auswertbares Röntgenbild zu erhalten, von der Korkplatte abgenommen werden, da sich eine solche Platte sich nicht homogen mit Röntgenstrahlen durchleuchten lässt. Diese wirft Schatten oder kann hellere Bereiche oder Linien in Röntgenbild erzeugen.

Bekannt geworden ist aus US 4 993 056 A ein Tablett zum Transport und zur Aufbewahrung von Gewebeproben, bei dem die Gewebeprobe zwischen zwei Platten eines Tablett festgeklemmt wird. Die eine Platte trägt ein röntgenopakes Gitter als Koordinatensystem. Bei einem solchen Tablett ist eine Nachjustierung der Gewebeprobe nicht möglich. Dieses Tablett ist aufgrund des relativ großen Herstellaufwandes als Mehrwegtablett konzipiert und muss nach jeder Benutzung aufwändig gereinigt und desinfiziert werden. Zudem können bei Verwendung eines solchen Tabletts Röntgenaufnahmen nicht in vertikaler Richtung gemacht werden. Auch ist das Zusammenpressen einer Gewebeprobe zwischen zwei Platten für einen Transport über längere Strecken ungeeignet. Ein Transport in Formalin ist mit diesem Tablett nicht möglich, das in Folge des Zusammenpressens der Probe diese mit dem Formalin nicht ausreichend in Kontakt kommt.

In WO 02/059571 A2 ist eine mehrteilige Anordnung für den Transport und/oder die Aufbewahrung von Gewebeproben bekannt. Diese Anordnung umfasst zwei manuell für die Zwecke der Aufbewahrung einer Gewebeprobe miteinander zu verbindende Teile. Die beiden Teile fassen einen Hohlraum ein, in dem die Gewebeprobe angeordnet ist. Zum Fixieren der Gewebeprobe beim Zusammenfügen der beiden Teile trägt eines der Teile in regelmäßigen Abständen nadelartige Spitzen. Zum Anpassen der beiden miteinander zu verbindenden Teile für das Aufbewahrungsbehältnis der Gewebeprobe können diese zurechtgeschnitten werden. Die in diesem Dokument notwendige Handhabung ist aufwendig. Überdies besteht für die mit den zusammenzusetzenden Teilen befassten Personen eine Verletzungsgefahr an den nagelartigen Spitzen.

Ausgehend von dem vordiskutierten Stand der Technik liegt der Erfindung daher die Aufgabe zugrunde, eine eingangs genannte Anordnung zu modifizieren, um eine kostengünstige Anordnung zu schaffen, wobei die Handhabung der Gewebeprobe vereinfacht und die Verletzungsgefahr verringert wird.

Diese Aufgabe wird erfindungsgemäß durch eine eingangs genannte, gattungsgemäße Anordnung gelöst, bei der
- das Tablett zumindest zweilagig aufgebaut ist und eine Deckschicht mit einer zum Anbringen der Gewebeprobe vorgesehenen Oberseite sowie einen mit der Deckschicht verbundenen Tragekörper aufweist,
- der zumindest eine Fixerstift über eine in Stechrichtung hinterschnittene Spitze verfügt, deren Hinterschnitt zur Verriegelung des Fixierstiftes an dem Tablett an der Innenseite der Deckschicht nach Einstechen desselben in das Tablett anliegt,
- wobei durch den Trageköper zumindest in dem Bereich eines vorgesehenen Einstechpunktes eines Fixierstiftes der Spitze beim Einstechen ein geringerer Einstechgegendruck entgegenwirkt als beim Einstechen der Deckschicht, und
- das Tablett und/oder der jeweilige Fixierstift zur Ausbildung einer Verriegelungsfalle über Mittel zum Begrenzen der Einstechtiefe des Fixierstiftes in das Tablett verfügen.

Bei dieser Anordnung für den Transport und/oder die Aufbewahrung einer menschlichen oder tierischen Gewebeprobe erfolgt die Fixierung einer chirurgisch entnommenen Gewebeprobe auf dem Tablett unter Verwendung bestimmter Fixierstifte. Das Tablett selbst ist zweilagig aufgebaut und umfasst eine obere Deckschicht, deren Oberseite die zum Anbringen der Gewebeprobe vorgesehene Seite ist, sowie einen darunter befindlichen und mit der Deckschicht verbundenen Tragekörper. Dabei ist vorgesehen, dass die Deckschicht selbst und der darunter befindliche Tragekörper einem darin zu verankernden Fixierstab jeweils einen unterschiedlichen Einstechgegendruck bereitstellen, wobei der Einstechgegendruck zum Hindurchstechen des Fixierstiftes mittels seiner Spitze durch die Deckschicht größer ist als der durch den Tragekörper bereitgestellte Einstechgegendruck. In Zusammenwirkung mit diesem Einstechgegendruckkontrast zwischen der Deckschicht und Tragekörper ist es möglich, den Fixierstift an dem Tablett zu verankern, und zwar durch Vorsehen einer hinterschnittenen Spitze, die sodann durch ihren Hinterschnitt einen oder mehrere Widerhaken bereitstellt. Die Spitze des Fixierstiftes wird vollständig durch die Deckschicht hindurchgestoßen, was zum Verankern eines Fixierstiftes an dem Tablett bestimmungsgemäß der Fall ist, wobei die Widerhaken bzw. der Hinterschnitt einer Verriegelung des Fixierstiftes an der Deckschicht des Tabletts dienen. Mithin liegen zur Verriegelung eines Fixierstiftes an dem Tablett der oder die Widerhaken an der Innenseite der Deckschicht an. Die Deckschicht kann durch die eigene materialbedingte Rückstellkraft nach dem Durchstechen der Spitze in den Hinterschnitt gebracht werden. Bevorzugt ist jedoch eine Ausgestaltung, bei der Fixierstift eine flache Spitze aufweist, sodass der zumindest eine Widerhaken in radialer Richtung abragt und nach Drehen des Fixierstiftes dieser einen durch den Einschnitt unverletzten Deckschichtabschnitt hintergreift. Eine solche Verriegelung ist nach Art einer Bajonettverriegelung konzipiert. Der Tragekörper des Tabletts ist ausgebildet, damit im Falle einer solchen, bajonettähnlichen Verriegelung des Fixierstiftes dieser durch eine Elastizität des Tragekörpers nicht in seine Einstechlage zurückgestellt wird.

Damit ein Fixierstift nicht durch das Tablett hindurchgestoßen werden kann, verfügt entweder das Tablett oder der jeweilige Fixierstift über Mittel zum Begrenzen der Einstechtiefe des Fixierstiftes. Als derartiges Einstechtiefenbegrenzungsmittel kann beispielsweise der Fixierstift mit Abstand zu dem durch die Spitze gebildeten Hinterschnitt eine oder mehrere, in radialer Richtung wirkende Ausladungen mit einem zur Spitze weisenden Anschlag aufweisen. Bei dem Vorgang des Durchstechens der Spitze des Fixierstiftes durch die Deckschicht wirkt diese Ausladung als Anschlag, durch den grundsätzlich ein weiteres Einstechen unter Anwendung der bestimmungsgemäßen Kräfte verhindert ist. Bei Vorsehen einer flachen Spitze bei einem solchen Fixierstift ist die Ausladung vorzugsweise ebenfalls flach ausgestaltet, wobei vorgesehen ist, dass die Ebene der zumindest einen Ausladung winklig zur Ebene der Spitze angeordnet ist. Sodann trifft die Einstechtiefenbegrenzungsausladung auf die Oberseite der Deckschicht quer zu dem Einstich durch die Spitze. Als Einstechtiefenbegrenzungsmittel kann anstelle oder auch zusätzlich zu der Konzeption einer oder mehrerer radialer Ausladungen bei den verwendeten Fixierstiften das Tablett eine untere, von der Spitze des Fixierstiftes nicht, zumindest nicht ohne Weiteres, durchdringbare Deckschicht aufweisen.

Als Tragekörper kann letztendlich jeder Körper dienen, der die vorstehend genannten Eigenschaften aufweist. Es ist daher grundsätzlich möglich, die Deckschicht auf einen kassetten- oder rahmenartig aufgebauten Tragekörper anzubringen, wobei der Bereich des oder der vorgesehenen Einstiche zur Fixierung der Fixierstifte an dem Tablett in einem rahmenlosen Bereich vorgesehen ist. Ein solcher Rahmen dient letztendlich zur Bereitstellung eines Verriegelungshohlraumes sowie zum Aussteifen der Deckschicht.

Für den Fall, dass das Tablett röntgengeeignete Eigenschaften aufweist, und zwar solche, dass dieses, ohne Schattenwürfe oder dergleichen zu erzeugen, durchleuchtet werden kann, hat sich überraschend gezeigt, dass der Einsatz einer handelüblichen PUR-Hartschaumplatte für diese Zwecke besonders eignet ist. Eine solche Hartschaumplatte verleiht dem Tablett nicht nur die notwendige Stabilität und gewährleistet ein bestimmungsgemäßes Verankern des oder Fixierstifte an der darauf angebrachten Deckschicht, sondern, wohl aufgrund der Homogenität des Materials und der vorgesehenen Dicke desselben, kann dieses in Bezug auf eine röntgenografische Abbildung der Gewebeprobe, beispielsweise des Tumors, ohne die Abbildungsqualität zu beeinträchtigen, eingesetzt werden. Ferner hat sich wiederum überraschend gezeigt, dass bei Verwendung einer solchen Platte als Tragekörper eines Tablett auch andere bildgebende Untersuchungen, wie beispielsweise Ultraschalluntersuchungen, an der auf dem Tablett befestigten Gewebeprobe durchgeführt werden können, ohne dass das Tablett das Ergebnis beeinträchtigt und daher die Gewebeprobe für derartige Untersuchungen nicht von dem Tablett abgenommen werden muss.

Eine Anordnung für den Transport und/oder die Aufbewahrung einer menschlichen oder tierischen Gewebeprobe wie vorbeschrieben lässt sich kostengünstig herstellen. So kann das Tablett beispielsweise aus einer für andere Zwecke konzipierten Sandwichplatte mit einem PUR-Hartschaumkern und beidseitiger kunststoffbeschichteter Zellstoffkarton-Deckschicht bestehen. Die Fixierstifte bestehen vorzugsweise aus einem Kunststoff, insbesondere aus einem faserverstärkten Kunststoff und sind typischerweise im Wege eines Spritzgussverfahrens hergestellt. Die Herstellungskosten einer solchen Anordnung können daher als relativ gering eingestuft werden mit der Folge, dass sich eine solche Anordnung insbesondere zum Herstellen von Einmalanordnungen bzw. Wegwerfanordnungen eignet. Dieses hat zudem den Vorteil, dass die gesamte Anordnung mit der darauf fixierten Gewebeprobe insgesamt entsorgt werden kann. Ein Entfernen derselben zur Weiterverwendung des Tabletts einschließlich einer entsprechenden Desinfektion kann daher entfallen.

Bei der beschriebenen Anordnung ist es ohne Weiteres möglich, die Oberseite der Deckschicht mit Kennzeichnungen zu versehen, beispielsweise solchen, die die Orientierung der Gewebeprobe im menschlichen oder tierischen Körper anzeigen. Derartige Markierungen sind vorzugsweise röntgenografisch opak ausgestaltet, damit diese im Röntgenbild sichtbar sind. Derartige Kennzeichnungen können beispielsweise durch Aufdrucken einer Beschriftung mit einem röntgenografisch opaken Druckmittel oder auch durch Applizieren von Kennzeichnungselementen erstellt werden. Sind Kennzeichnungselementen vorgesehen, können diese ebenfalls als Kunststoffspritzgussteile konzipiert sein, die auf die Deckschicht aufgeklebt sein können. Auch ist es möglich, an deren Rückseite eines solchen Kennzeichnungselements ein oder mehrere Verriegelungsfortsätze anzuordnen. Eine Fixierung an dem Tablett erfolgt sodann durch einfaches Eindrücken derselben in die Deckschicht des Tabletts. Bei einer solchen Ausgestaltung ist es möglich, das Tablett mit den entsprechenden Kennzeichnungen vormontiert einem Operateur zur Verfügung zu stellen oder dem Operateur die Möglichkeit zu lassen, selbst die Orientierungskennzeichnungen nach Fixieren der Gewebeprobe auf dem Tablett auf dem Tablett anzubringen.

Nachfolgend ist die Erfindung anhand eines Ausführungsbeispiels unter Bezugnahme auf die beigefügten Figuren beschrieben. Es zeigen:
- **Fig. 1:**: in einer schematisierten perspektivischen Ansicht ein Tablett für eine Anordnung für den Transport und/oder die Aufbewah- rung einer menschlichen oder tierischen Gewebeprobe,
- **Fig. 2:**: eine Seitenansicht eines Fixierstiftes zum Fixieren einer Ge- webeprobe auf dem Tablett der Figur 1,
- **Fig. 2a:**: den handhabungsseitigen Ausschnitt eines weiteren Fixierstif- tes zum Fixieren einer Gewebeprobe auf dem Tablett der Fi- gur 1,
- **Fig. 3:**: eine vergrößerte Schnittdarstellung durch den Fixierstift der Figur 2 entlang der Linie A - B,
- **Fig. 4:**: einen schematisierten Teilschnitt durch eine mittels Fixierstif- ten gemäß Figur 2 auf dem Tablett der Figur 1 fixierte Gewe- beprobe in einem Ausschnitt und
- **Fig. 5:**: die Anordnung der Figur 4 aufgeständert in einem Ständer.

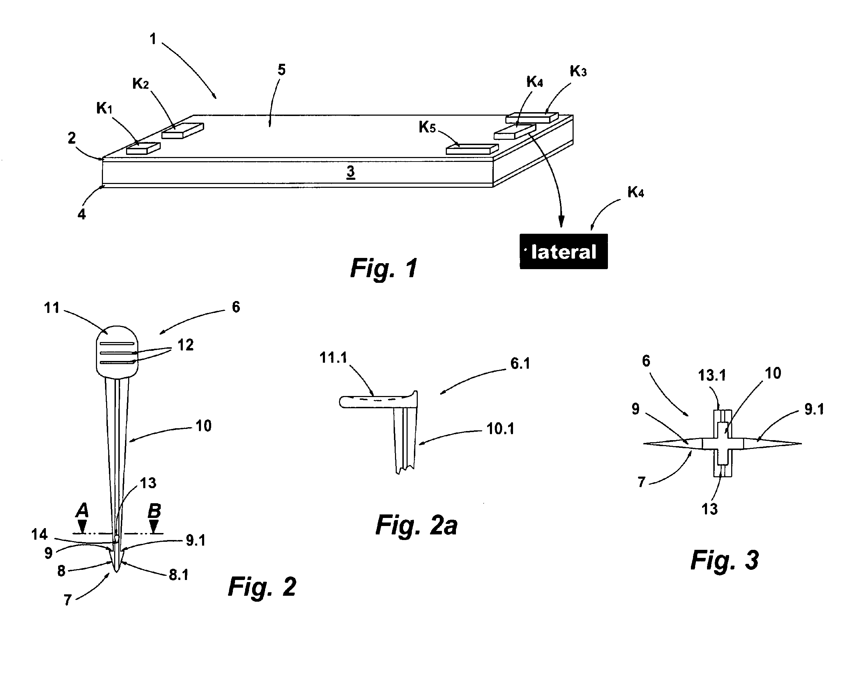

Ein Tablett 1 ist Teil einer Anordnung für den Transport und/oder die Aufbewahrung einer menschlichen oder tierischen Gewebeprobe. Das Tablett 1 des dargestellten Ausführungsbeispiels ist als Sandwichkörper aufgebaut und verfügt über eine obere Deckschicht 2, eine Polyurethan-Hartschaumplatte als Tragekörper 3 und über eine untere Deckschicht 4. Bei den beiden Deckschichten 2, 4 handelt es sich um kunststoffbeschichtete Zellstoffkartonschichten in einer Stärke von etwa 0,5 mm. Bei dem Tragekörper 3 handelt es sich um eine handelsübliche PUR-Hartschaumplatte mit einer Geschlossenzelligkeit von mehr als 95 %. Die Porengröße des Hartschaums ist gering und überschreitet vorzugsweise eine durchschnittliche Porengröße von 0,2 bis 0,4 mm nicht. Die Eigenschaft des Hartschaums weist nur eine geringe elastische Rückstellrate auf. Der Hartschaum ist ohne Weiteres einschneidbar. Die Dicke des in Figur 1 dargestellten Tabletts beträgt 10 mm.

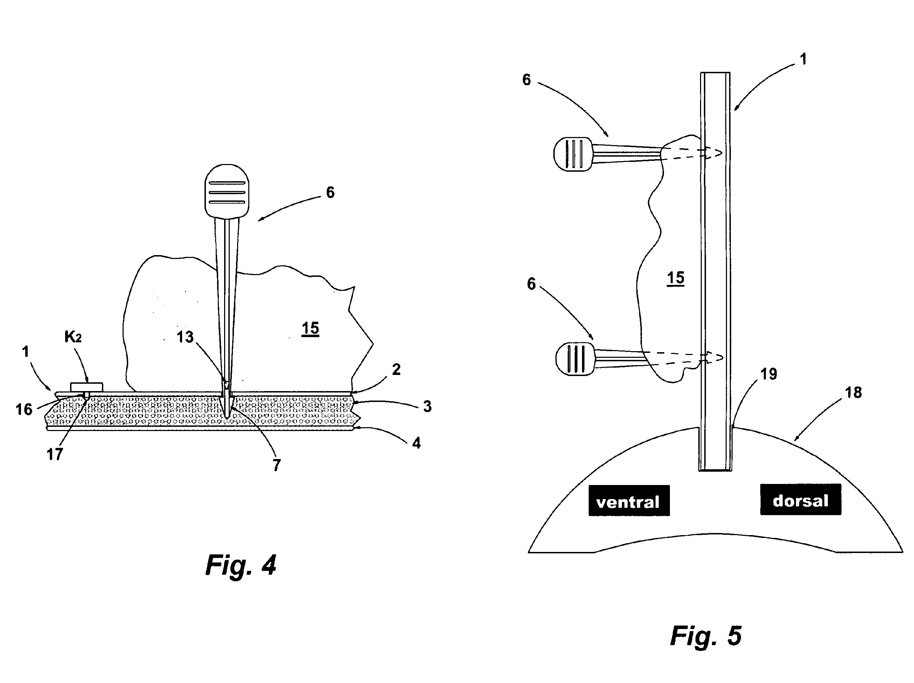

Die Oberseite 5 der oberen Deckschicht 2 des Tabletts 1 dient zum Fixieren einer Gewebeprobe. Zum Kennzeichnen der Orientierung der auf der Oberseite 5 des Tabletts 1 anzubringenden Gewebeprobe sind auf der Oberseite 5 der oberen Deckschicht 2 mehrere Kennzeichnungen K₁ - K₅ angebracht. Bei der Kennzeichnung K₁ handelt es sich um ein "L" bzw. ein "R", stehend für rechts ("R") bzw. links ("L"). Die Kennzeichnungen K₂ - K₅ geben die Orientierung der entnommenen Gewebeprobe innerhalb des Körpers wieder. Daher steht bei dem dargestellten Ausführungsbeispiel die Kennzeichnung K₂ für "medial", die Kennzeichnung K₃ für "cranial", die Kennzeichnung K₄ hier für "lateral" und das Kennzeichnen für K₅ für "caudal". Entsprechend dieser, bei dem dargestellten Ausführungsbeispiel vorgegebenen Orientierung, wird die Gewebeprobe auf der Oberseite 5 des Tabletts 1 fixiert. Die Kennzeichnungen K₁ - K₅ sind Kunststoffteile, die wie zu Figur 4 beschrieben, an dem Tablett 1 befestigt sind, und in die die jeweilige Orientierung als Aussparung eingebracht ist. Das Kunststoffmaterial der Kennzeichnungen K₁ - K₅ ist röntgenopak, so dass die darin eingebrachte Schrift im Röntgenbild erkennbar ist. Im Wege eines Beispiels ist die Kennzeichnung K₄ in Figur 1 zusätzlich in einer Draufsicht gezeigt. Die Kennzeichnungen K₁ - K₅ sind, wie in Figur 1 dargestellt außerhalb des Bereiches der Oberseite 5 des Tabletts1 angeordnet, in der die Gewebeprobe platziert werden soll. Von Vorteil ist, die Kennzeichnungen K₁ - K₅ zudem dergestalt anzubringen, dass eine auf dem Tablett 1 fixierte Gewebeprobe auch in cranio-caudaler Richtung geröntgt werden kann, ohne dass Kennzeichnungen K₁ - K₅ als Schatten erkennbar sind. Aus diesem Grunde sind die Kennzeichnungen K₃ und K₅ entlang der rechten Seite des Tabletts 1 und somit außerhalb der genannten Röntgenrichtung angeordnet.

Infolge der eingesetzten Materialien und den Sandwichaufbau ist das Tablett 1 hinreichend formstabil. Gleichfalls ist das Tablett 1 beständig gegenüber über Formalin, welches zur Aufbewahrung von Gewebeproben benötigt wird.

Zum Fixieren einer Gewebeprobe auf dem Tablett 1 werden mehre Fixierstifte verwendet. Figur 2 zeigt in einer Seitenansicht eine Ausgestaltung eines solchen Fixierstiftes 6. Der Fixierstift 6 ist ein Kunststoffspritzgussteil und besteht aus einem Hartkunststoff, der bei dem dargestellten Ausführungsbeispiel faserverstärkt ist. Der Fixierstift 6 verfügt über eine flache Spitze 7, die zum Durchstechen derselben durch eine Gewebeprobe und zum Durchstechen der oberen Deckschicht 2 des Tabletts 1 über zwei Schneiden 8, 8.1 verfügt. Die Spitze 7 ist hinterschnitten. Die den Hinterschnitt bildenden Kanten der Spitze 7 sind in Figur 2 mit dem Bezugszeichen 9, 9.1 gekennzeichnet. An die Spitze 7 ist ein Schaft 10 angeformt. Der Schaft 10 weist eine kreuzförmige Querschnittsfläche auf, wie dieses aus der Schnittdarstellung der Figur 3 erkennbar ist. Der Schaft 10 ist insgesamt konisch, sich in Richtung zur Spitze 7 hin verjüngend, konzipiert. An das obere Ende des Schaftes 10 ist eine Handhabe 11 angeformt. Die Flachseiten der Handhabe 11 sind durch Wülste 12 zur Erhöhung der Griffigkeit der Handhabe 11 gekennzeichnet. Bei dem dargstellten Ausführungsbeispiel folgt die Ausrichtung der Handhabe 11 der Längserstreckung des Schaftes 10. Figur 2a zeigt eine alternative Ausgestaltung eines Fixierstiftes 6.1 dargestellt, bei dem die Handhabe 11.1 rechtwinklig zum Schaft 10.1 angeordnet ist und von diesem in einer Richtung abragt. Die Handhabe 11.1 weist oberseitig eine Einmuldung auf (gestrichelt). Diese Handhabe 11.1 kann durch Aufbringen der notwendigen Eindrückkraft mit dem Daumen durch die zu fixierende Gewebeprobe in das Tablett eingedrückt werden. Eine weitere, in den Figuren nicht dargestellte Ausgestaltung betrifft eine Anordnung der Handhabe zum Schaft, bei der die Handhabe nach Art eines Flachkopfes zentrisch auf dem Schaft des Fixierstiftes sitzt.

Mit Abstand von der Spitze 7 sind an den Schaft 10 in radialer Richtung zwei, einander diametral zur Längsachse des Schaftes 10 gegenüberliegende Ausladungen 13, 13.1 angeformt. Die Ausladungen 13, 13.1 dienen als Einstecktiefenbegrenzungsmittel des Fixierstiftes 6 zum Begrenzen der Einstechtiefe der Spitze 7 in das Tablett 1. Die Spitze 7 ist, wie aus der Querschnittsdarstellung der Figur 3 erkennbar, als flache Spitze ausgeführt. Die Ebene der Ausladungen 13, 13.1 ist rechtwinklig zur Ebene der Spitze 7 angeordnet. Die Ausladungen 13, 13.1 sind ihrerseits in Richtung zur Spitze 7 weisend verjüngt und verfügen unterseitig über eine stumpfe Anschlagkante 14. Die Weite der Ausladungen 13, 13.1 in radialer Richtung ist abgestimmt darauf, dass durch diese ein Durchstechen durch die zu fixierende Gewebeprobe nicht oder nicht merklich behindert ist, dass gleichwohl diese einen wirksamen Anschlag beim Vorgang des Einstechens des Fixierstiftes 6 in die obere Deckschicht 2 des Tabletts 1 darstellen.

Fixiert wird eine Gewebeprobe auf dem Tablett 1, typischerweise mit zumindest zwei oder auch mehreren Fixierstiften. Für den Fall, dass eine Gewebeprobe auf dem Tablett 1 orientiert fixiert werden soll, was die Regel sein dürfte, ist eine Fixierung mit zumindest zwei Fixierstiften 6 notwendig. Die Fixierstifte können eine unterschiedliche Länge ihres Schaftes aufweisen, so dass in Abhängigkeit von der Dicke der zu befestigenden Gewebeprobe längere oder auch kürzere Fixierstifte zum Fixieren derselben auf dem Tablett eingesetzt werden können.

Eine auf dem Tablett 1 mit mehreren Fixierstiften 6 fixierte Gewebeprobe 15 ist in einer Teilschnittdarstellung in Figur 4 gezeigt. Nachdem zunächst die Gewebeprobe 15 hinsichtlich ihrer Orientierung in dem Körper, aus dem sie entnommen worden ist, auf die Oberseite 5 der oberen Deckschicht 2 des Tabletts 1 aufgelegt worden ist, ist der in Figur 4 erkennbare Fixierstift durch den Rand der Gewebeprobe 15 hindurchgesteckt und mit seiner Spitze 7 durch die obere Deckschicht 2 hindurch und in den durch die PUR-Hartschaumplatte gebildeten Tragekörper 3 eingestochen worden. Begrenzt worden ist der Einstechvorgang durch die winkelversetzt zu den Schneiden 8, 8.1 der Spitze 7 angeordneten Ausladungen 13, 13.1, als diese auf der Oberseite 5 der oberen Deckschicht 2 als Anschlag zur Wirkung zur Anlage kommen. Die winkelversetzte Anordnung der Ausladungen 13, 13.1 zu der Ebene der Spitze 7 ist von Vorteil, da die Ausladungen 13, 13.1 auf einem durch die Schneiden 8, 8.1 unverletzten Abschnitt der oberen Deckschicht zur Anlage gelangen und somit wirksam den Einstechvorgang beenden. Durch die als Anschlag dienenden Ausladungen 13, 13.1 wird während des Einstechvorganges der dem die Gewebeprobe fixierenden Operateur durch eine plötzliche Erhöhung des Einstechgegendruckes eine haptische Rückmeldung übermittelt, dass die bestimmungsgemäße Einstechtiefe des Fixierstiftes 6 in das Tablett 1 erreicht ist. Die Länge der Spitze 7 ist kürzer bemessen als die Dicke des Tragekörpers 3. Der Abstand der Ausladungen 13, 13.1 von dem Hinterschnitt 9, 9.1 der Spitze 7 ist ausreichend, damit die obere Deckschicht 2 in die durch den Hinterschnitt 9, 9.1 und die Ausladungen 13, 13.1 gebildete Verriegelungsfalle einpassen kann. Typischerweise wird man unter Berücksichtigung der Dicke der Deckschicht 2 einen entsprechenden Abstand zum Bereitstellen eines gewissen Spiels vorsehen. Dabei ist vorgesehen, dass der Abstand der Anschlagkanten der Ausladungen 13, 13.1 von dem vorderen Ende der Spitze 7 kleiner als die Dicke des Tragekörpers 3 ist.

Nach Einbringen der Spitze 7 in den Tragekörper 3 wird der Fixierstift 6 um etwa 90° gedreht, damit die den Hinterschnitt bildenden Kanten 9, 9.1 zum Verriegeln des Fixierstiftes 6 an der dem Tablett 1 gegen durch die Schneiden 8, 8.1 im Zuge des Einstechens unverletzte Abschnitte der Oberseite der oberen Deckschicht 2 wirken. Das Einstechen der Spitze 7 und das Durchstechen derselben durch die obere Deckschicht 2 und das anschließende Drehen der Ebene der Spitze 7 um ihre Längsachse aus ihrer Einstechlage in eine Verriegelungslage erlaubt es, die Verriegelung als bajonettähnlich anzusprechen. Figur 4 zeigt den Fixierstift 6 in der mit seiner Spitze 7 verriegelnden und gegenüber der Einstechorientierung um 90° gedrehten Lage.

Bei den Kennzeichnungen K₁ - K₅ handelt es sich um röntgenopake Kunststoffschilder, die jeweils einen oder mehrere Verriegelungsfortsätze 16 aufweisen, wie dieses anhand des Kennzeichnens K₂ in Figur 4 erkennbar ist. Die Verriegelungsfortsätze 16 umfassen einen Verriegelungskopf 17, der einen Hinterschnitt ausbildet, in den die obere Deckschicht 2 nach Durchstechen des Verriegelungsfortsatzes 16 durch die obere Deckschicht 2 aufgrund ihrer materialbedingten Rückstellkraft eindringt.

Teil der vorbeschriebenen Anordnung können ein oder mehrere Ständer zum Aufständern des Tabletts 1 mit einer darauf fixierten Gewebeprobe 15 sein. Ein solcher Ständer ist beispielhaft zusammen mit dem Tablett 1 und der darauf mit Fixierstiften 6 fixierten Gewebeprobe 15 gezeigt und ist mit dem Bezugszeichen 18 gekennzeichnet. Der Ständer 18 besteht letztendlich aus zwei parallel zueinander angeordneten gleichartigen Ständern, die jeweils oberseitig eine Aussparung 19 zum Einstecken eines Randabschnittes des Tabletts 1 aufweisen. Die Ständer 18 dienen zum Aufstellen des Tabletts 1, für den Fall, dass radiologische Untersuchungen an der Gewebeprobe 15 in einer bestimmten, von der Horizontalen abweichenden Raumlage durchgeführt werden sollen. Bei dem dargestellten Ausführungsbeispiel der Ständer 18 ist die Aussparung 19 rechtwinklig zur Horizontalen ausgeführt. Es können unterschiedliche Ständer vorgesehen sein, die in unterschiedlichem Maße gegenüber der Horizontalen geneigte Aussparungen aufweisen, beispielsweise Aussparungen mit einer Neigung von 30, 45 oder 60°. Der Ständer 18 kann aus demselben Sandwichplattenmaterial bestehen wie das Tablett. Vorzugsweise sind die Ständer 18 beschriftet, um die Orientierung der Gewebeprobe erkennen zu können. Hierzu eignet sich eine Beschriftung "ventral" (vorne) und "dorsal" (hinten). Die diesbezüglichen Kennzeichnungen können bei Verwendung eines Ständers aus einem Material wie das Tablett wie die Kennzeichnungen K₁ - K₅ konzipiert sein.

Das Tablett 1 sowie die Fixierstifte 6 sind kostengünstig herstellbar. Dabei handelt es sich bei der vorbeschriebenen Anordnung für den Transport und/oder die Aufbewahrung einer menschlichen oder tierischen Gewebeprobe um eine typischerweise nur einmal verwertbare Anordnung, so dass diese bei nicht mehr benötigter Gewebeprobe zusammen mit dieser entsorgt wird.

Die Eigenschaften des beschriebenen Tabletts, insbesondere unter Verwendung der als Tragekörper 3 eingesetzten PUR-Hartschaumschicht bzw. -platte und der beschriebenen Verriegelung der Gewebeproben auf der Oberseite der oberen Deckschicht, gestattet es, das Tablett 1 ebenfalls als Schwimmkörper einzusetzen und die Gewebeprobe in einer Überkopfanordnung, in Formalin eingetaucht, aufzubewahren. Dies ermöglicht ein sauberes Einlegen und Herausnehmen der Gewebeprobe aus einem Formalinbad. Zu diesem Zweck kann die untere Deckschicht 4 über laschenartige Fortsätze verfügen, die aufgebogen als Handhabe zum Einlegen und Herausnehmen des Tabletts 1 mit der darauf fixierten Gewebeprobe 15 in ein Formalinbad in einer Überkopfanordnung, bei der die Gewebeprobe zuunterst und das Tablett 1 zuoberst angeordnet sind, dienen.

Die vorstehende Beschreibung der Erfindung stellt eine beispielhafte Ausgestaltung der Erfindung dar. Ohne den Umfang der Ansprüche zu verlassen, erschließen sich für einen auf dem Gebiet tätigen Fachmann unter Berücksichtigung dieser Ausführungen zahlreiche weitere Ausgestaltungen.

### Bezugszeichenliste

- 1: Tablett
- 2: obere Deckschicht
- 3: Tragekörper
- 4: untere Deckschicht
- 5: Oberseite
- 6, 6.1: Fixierstift
- 7: Spitze
- 8, 8.1: Schneide
- 9, 9.1: Hinterschnitt
- 10, 10.1: Schaft
- 11, 11.1: Handhabe
- 12: Wulst
- 13, 13.1: Ausladung
- 14: Anschlagkante
- 15: Gewebeprobe
- 16: Verriegelungsfortsatz
- 17: Verriegelungskopf
- 18: Ständer
- 19: Aussparung

- K₁ - K₅: Kennzeichnung

## Patentansprüche

1. Anordnung für den Transport und/oder die Aufbewahrung einer menschlichen oder tierischen Gewebeprobe (15), umfassend ein Tablett (1) und ein oder mehrere zur Fixierung der Gewebeprobe (15) auf dem Tablett (1) dienende, jeweils eine Spitze (7) aufweisende und mit dieser in das Tablett (1) einstechbare Fixierstifte (6, 6.1), **dadurch gekennzeichnet, dass**
- das Tablett (1) zumindest zweilagig aufgebaut ist und eine Deckschicht (2) mit einer zum Anbringen der Gewebeprobe (15) vorgesehenen Oberseite (5) sowie einen mit der Deckschicht (2) verbundenen Tragekörper (3) aufweist,
- der zumindest eine Fixerstift (6, 6.1) über eine in Stechrichtung hinterschnittene Spitze (7) verfügt, deren Hinterschnitt (9, 9.1) zur Verriegelung des Fixierstiftes (6, 6.1) an dem Tablett (1) an der Innenseite der Deckschicht (2) nach Einstechen desselben in das Tablett (1) anliegt,
- wobei durch den Trageköper (3) zumindest in dem Bereich eines vorgesehenen Einstechpunktes eines Fixierstiftes (6, 6.1) der Spitze (7) beim Einstechen ein geringerer Einstechgegendruck entgegenwirkt als beim Einstechen der Deckschicht (2), und
- das Tablett und/oder der jeweilige Fixierstift (6, 6.1) zur Ausbildung einer Verriegelungsfalle über Mittel zum Begrenzen der Einstechtiefe des Fixierstiftes (6, 6.1) in das Tablett (1) verfügen.

2. Anordnung nach Anspruch 1, **dadurch gekennzeichnet, dass** der jeweilige Fixierstift (6, 6.1) von dem Hinterschnitt (9, 9.1) beabstandet wenigstens eine Ausladung (13, 13.1) in radialer Richtung aufweist.

3. Anordnung nach Anspruch 1 oder 2, **dadurch gekennzeichnet, dass** das Tablett (1) eine mit dem Tragekörper (3) verbundene, an der der Deckschicht (2) gegenüberliegenden Seite angeordnete zweite Deckschicht (4) aufweist.

4. Anordnung nach einem der Ansprüche 1 bis 3, **dadurch gekennzeichnet, dass** die Spitze (7) des Fixierstiftes (6, 6.1) als flache Spitze ausgeführt ist.

5. Anordnung nach Anspruch 4 in seinem Rückbezug auf Anspruch 2, **dadurch gekennzeichnet, dass** die wenigstens eine radiale Ausladung (13, 13.1) flach ausgebildet ist und die Ebene der Ausladung (13, 13.1) winklig, insbesondere rechtwinklig zur Ebene der Spitze (7) angeordnet ist.

6. Anordnung nach einem der Ansprüche 1 bis 5, **dadurch gekennzeichnet, dass** der zumindest eine Fixierstift (6, 6.1) ein Kunststoffteil, insbesondere ein faserverstärktes Kunststoffteil ist.

7. Anordnung nach einem der Ansprüche 1 bis 6, **dadurch gekennzeichnet, dass** der zumindest eine Fixierstift (6, 6.1) über eine Handhabe (11) zum Handhaben des Fixierstiftes (6, 6.1) verfügt, welche Handhabe (11) vorzugsweise an dem der Spitze (7) gegenüberliegenden Ende angeordnet ist.

8. Anordnung nach einem der Ansprüche 1 bis 7, **dadurch gekennzeichnet, dass** die Handhabe gegenüber der Längserstreckung des Schaftes des Fixierstiftes abgewinkelt ist.

9. Anordnung nach einem der Ansprüche 1 bis 8, **dadurch gekennzeichnet, dass** die Deckschicht (2) des Tabletts (1) eine, vorzugsweise kunststoffbeschichtete, Zellstoffkartonschicht ist.

10. Anordnung nach einem der Ansprüche 1 bis 9, **dadurch gekennzeichnet, dass** der Tragekörper (3) des Tabletts (1) als materialhomogene Trägerschicht ausgeführt ist.

11. Anordnung nach Anspruch 10, **dadurch gekennzeichnet, dass** die Trägerschicht eine Hartschaumschicht ist.

12. Anordnung nach Anspruch 11, **dadurch gekennzeichnet, dass** die Hartschaumschicht als PUR-Hartschaumschicht ausgeführt ist.

13. Anordnung nach einem der Ansprüche 1 bis 12, **dadurch gekennzeichnet, dass** die zum Anbringen der Gewebeprobe vorgesehene Deckschicht (2) auf ihrer Oberseite (5) Orientierungskennzeichnungen (K₁ - K₅) zum Orientieren und Befestigen der Gewebeprobe (15) auf dem Tablett (1 ) trägt.

14. Anordnung nach Anspruch 13, **dadurch gekennzeichnet, dass** die Orientierungskennzeichnungen (K₁ - K₅) in der Deckschicht (2) des Tabletts (1) verankerbare Kunststoffteile sind.

15. Anordnung nach Anspruch 13 oder 14, **dadurch gekennzeichnet, dass** die Orientierungskennzeichnungen (K₁ - K₅) im Röntgenbild sichtbar bzw. röntgenopak sind.

16. Anordnung nach einem der Ansprüche 1 bis 15, **dadurch gekennzeichnet, dass** der Anordnung ein oder mehrere Ständer (18) zum Aufstellen des Tabletts (1) zugeordnet sind.

## Claims

1. An arrangement for the transport and/or safekeeping of a human or animal tissue sample (15), comprising a tablet (1) and one or a plurality of fixing pins (6, 6.1) for fixing the tissue sample (15) to the tablet (1), each of said pins having a tip (7) for piercing the pins into the tablet (1), **characterised in that**
- the tablet (1) is designed with at least two layers and exhibits a cover layer (2) having an upper face (5) provided for applying the tissue sample (15) and a support member (3) connected to the cover layer (2),
- the at least one fixing pin (6, 6.1) comprises a tip (7) that is undercut in the direction of piercing and whose undercut (9, 9.1) sits against the inside of the cover layer (2) for locking the fixing pin (6, 6.1) on the tablet (1) after the pin pierces the tablet (1),
- wherein - at least within the region of a predetermined piercing point of a fixing pin (6, 6.1) - the tip (7) meets a lower piercing resistance when piercing the support member (3) than when it pierces the cover layer (2), and
- the tablet and/or the respective fixing pin (6, 6.1) comprise means for limiting the pierce depth of the fixing pin (6, 6.1) into the tablet (1) for forming a locking latch.

2. An arrangement according to Claim 1, **characterised in that** the respective fixing pin (6, 6.1) exhibits at least one radial projection (13, 13.1) spaced apart from the undercut (9, 9.1).

3. An arrangement according to Claim 1 or 2, **characterised in that** the tablet (1) exhibits a second cover layer (4) that is bonded to the support member (3) on the side opposite the cover layer (2).

4. An arrangement according to any one of Claims 1 to 3, **characterised in that** the tip (7) of the fixing pin (6, 6.1) is configured as a flat tip.

5. An arrangement according to Claim 4 in its reference back to Claim 2, **characterised in that** the at least one radial projection (13, 13.1) is flat in configuration and that the plane of the projection (13, 13.1) is arranged at an angle, in particular at a right angle, to the plane of the tip (7).

6. An arrangement according to any one of Claims 1 to 5, **characterised in that** the at least one fixing pin (6, 6.1) is a plastic part, in particular a fiber-reinforced plastic part.

7. An arrangement according to any one of Claims 1 to 6, **characterised in that** the at least one fixing pin (6, 6.1) has a handle (11) for handling the fixing pin (6, 6.1), said handle (11) being preferentially disposed at the end that is opposite the tip (7).

8. An arrangement according to any one of Claims 1 to 7, **characterised in that** the handle is angled relative to the longitudinal extension of the shaft of the fixing pin.

9. An arrangement according to any one of Claims 1 to 8, **characterised in that** the cover layer (2) of the tablet (1) is a cellulose cardboard layer, that is preferably plastic-coated.

10. An arrangement according to any one of Claims 1 to 9, **characterised in that** the support member (3) of the tablet (1) is executed as a materially homogeneous support layer.

11. An arrangement according to Claim 10, **characterised in that** the support layer is a rigid foam layer.

12. An arrangement according to Claim 11, **characterised in that** the rigid foam layer is a PUR rigid foam layer.

13. An arrangement according to any one of Claims 1 to 12, **characterised in that** the cover layer (2) provided for applying the tissue sample carries on its upper face (5) orientation marks (K₁ - K₅) for orienting and attaching the tissue sample (15) on the tablet (1).

14. An arrangement according to Claim 13, **characterised in that** the orientation marks (K₁ - K₅) are plastic parts that can be anchored in the cover layer (2) of the tablet (1).

15. An arrangement according to Claim 13 or 14, **characterised in that** the orientation marks (K₁ - K₅) are x-ray-opaque in the x-ray image.

16. An arrangement according to any one of Claims 1 to 15, **characterised in that** one or a plurality of stands (18) for setting up the tablet (1) are associated with the arrangement.

## Revendications

1. Ensemble de transport et/ou de conservation d'un échantillon tissulaire (15) humain ou animal, comprenant une tablette (1) et une ou plusieurs tiges de fixation (6, 6.1) servant à fixer l'échantillon tissulaire (15) sur la tablette (1), présentant chacune une pointe (7) pour être piquée grâce à celle-ci dans la tablette (1) **caractérisé en ce que**
- la tablette (1) est au moins structurée en deux couches et présente une couche de couverture (2) avec une face supérieure (5) prévue pour mettre en place l'échantillon tissulaire (15) ainsi qu'un corps porteur (3) associé à la couche de couverture (2),
- l'au moins une tige de fixation (6, 6.1) dispose d'une pointe (7) contre-découpée dans le sens de piquage, laquelle contre-découpe (9, 9.1) est, afin d'immobiliser la tige de fixation (6, 6.1) après son piquage sur la tablette (1), en contact contre la face intérieure de la couche de couverture (2),
- par rapport à la contre-pression exercée lors du piquage dans la couche de couverture (2), une contre-pression de piquage plus faible s'exerçant sur la pointe (7) lors du perçage du corps porteur (3), du moins dans la zone prévue de piquage d'une tige de fixation (6,6.1),
- la tablette et/ou ladite tige de fixation (6, 6.1) dispose, afin de former un loquet d'immobilisation, de moyens destinés à limiter la profondeur de piquage d'une tige de fixation (6, 6.1) dans la tablette (1).

2. Ensemble selon la revendication 1 **caractérisé en ce que** ladite tige de fixation (6, 6.1) présente à distance de la contre-découpe (9, 9.1) au moins un montant (13, 13.1) dans le sens radial.

3. Ensemble selon la revendication 1 ou 2 **caractérisé en ce que** la tablette (1) présente une seconde couche de couverture (4) reliée au corps porteur (3), disposée sur la face opposée à la couche de couverture (2).

4. Ensemble selon l'une des revendications 1 à 3 **caractérisé en ce que** la pointe (7) de la tige de fixation (6, 6.1) est conformée en pointe plate.

5. Ensemble selon la revendication 4 en référence à la revendication 2 **caractérisé en ce qu'**au moins un montant (13.13.1) radial est conformé de façon plate et que le plan du montant (13, 13.1) est disposé en angle, notamment en angle droit par rapport au plan de la pointe (7).

6. Ensemble selon l'une des revendications 1 à 5 **caractérisé en ce que** l'au moins une tige de fixation (6, 6.1) est une pièce en plastique, notamment une pièce en plastique renforcée en fibres de verre.

7. Ensemble selon l'une des revendications 1 à 6 **caractérisé en ce que** l'au moins une tige de fixation (6, 6.1) dispose d'une tête de préhension (11) pour manipuler la tige de fixation (6, 6.1), laquelle tête de préhension (11) étant disposée de préférence à l'extrémité opposée à la pointe (7).

8. Ensemble selon l'une des revendications 1 à 7 **caractérisé en ce que** la tête de préhension est disposée en angle par rapport à l'étendue longitudinale de la tige de ladite tige de fixation.

9. Ensemble selon l'une des revendications 1 à 8 **caractérisé en ce que** la couche de couverture (2) de la tablette (1) est une couche de carton cellulaire de préférence recouverte de matière plastique.

10. Ensemble selon l'une des revendications 1 à 9 **caractérisé en ce que** le corps porteur (3) de la tablette (1) est configuré en couche porteuse à matériau homogène.

11. Ensemble selon la revendication 10 **caractérisé en ce que** la couche porteuse est une couche en mousse rigide.

12. Ensemble selon la revendication 11 **caractérisé en ce que** la couche en mousse rigide est configurée en mousse de polyuréthane.

13. Ensemble selon l'une des revendications 1 à 12 **caractérisé en ce que** la couche de couverture (2) prévue pour mettre en place l'échantillon tissulaire comporte sur sa face supérieure (5) des marquages d'orientation (K₁ - K₅) afin d'orienter et de fixer l'échantillon tissulaire (15) sur la tablette (1).

14. Ensemble selon la revendication 13 **caractérisé en ce que** les marquages d'orientation (K₁ - K₅) dans la couche de couverture (2) de la tablette (1) sont des pièces de plastique ancrables.

15. Ensemble selon la revendication 13 ou 14 **caractérisé en ce que** les marquages d'orientation (K₁ - K₅) sont visibles sur une radio ou un cliché radiographique.

16. Ensemble selon l'une des revendications 1 à 15 **caractérisé en ce qu'**un ou plusieurs présentoirs (18) destinés à mettre la tablette (1) debout sont affectés à l'ensemble.
